# EUROPEAN PATENT SPECIFICATION

(11) **EP 0 634 105 B1**
(45) Date of publication and mention of the grant of the patent: **06.05.1999**
(21) Application number: 94305216.7
(22) Date of filing: 15.07.1994
(51) Int. Cl.: A23K 1/175, A23L 1/304, A61K 33/06, C01F 11/06

(54) **Calcium containing composition**
Calcium enthaltende Zusammensetzung
Composition contenant du calcium

(30) Priority: 16.07.1993 JP 176503/93; 21.04.1994 JP 83516/94; 28.04.1994 JP 91694/94
(43) Date of publication of application: 18.01.1995
(73) Proprietor: BIG BEAR BIO INC, San Mateo, California 94402 (US)
(72) Inventor: Kumagai, Yoshinari, Fujisawa-shi, Kanagawa (JP); Kubo, Azuma, Hirakata-shi, Osaka (JP)
(74) Representative: VOSSIUS & PARTNER

(56) References cited:
- PATENT ABSTRACTS OF JAPAN vol. 17, no. 554 (C-1118) 06 October 1993 & JP-A-05 161 480 (INOUE RANKO) 29 June 1993
- DATABASE WPI Week 8315, Derwent Publications Ltd., London, GB; AN 83-35346K & JP-A-58 036 348 (NISHIMURA K) 3 March 1983
- PATENT ABSTRACTS OF JAPAN vol. 4, no. 129 (C-024) 10 September 1980 & JP-A-55 081 552 (NISHIMURA KAZUNORI) 19 June 1980
- DATABASE WPI Week 7820, Derwent Publications Ltd., London, GB; AN 78-35941A & JP-A-53 038 593 (SHIMUZI S) 8 April 1978
- DATABASE WPI Week 9148, Derwent Publications Ltd., London, GB; AN 91-351106 & JP-A-3 236 750 (ABE T) 22 October 1991
- Translations of the five japanese documents cited in the european search report have been filed and included in the dossier (pages 44-77).

## Description

This invention relates to a calcium containing composition derived from the shells of sea urchins which gives high oral bioavailability; a manufacturing method for such a composition; a food containing such a composition; an animal feed containing such a composition, and pharmaceutical compositions containing such a composition.

Various types of calcium formulations and health foods which contain calcium as an active component have been proposed previously to supplement dietary calcium. The calcium used for such formulations and health foods is generally made from calcium carbonate derived from mineral origins such as limestone and lime milk, calcium phosphate, calcium hydroxide, calcium lactate, calcium gluconate, shellfish derived calcium such as oyster shells, other mollusc shells and corals, crustaceans such as crabs, and shrimps, seaweeds such as tangles, hijikia, and undaria, mammalian bones, and egg shells.

However, the calcium made from these materials generally does not give sufficient oral bioavailability. Furthermore, oral bioavailability of calcium in humans is generally extremely low unless it is administered with Vitamin D or proteins and it is difficult to supply the required amount of calcium to humans by itself. In addition, people in Japan take only 531 mg per day of calcium on average according to the National Nutrition Investigation Report of the Ministry of Health and Welfare. This level is less than a half of that in Western countries.

Under such circumstances, the inventors have tried to find a calcium containing composition having a high oral bioavailability. As a result, the invention was made by the discovery that a calcium containing composition with high oral bioavailability can be obtained, surprisingly, by baking sea urchin shells which have been disposed of as not having any use.

The present invention provides a calcium containing composition derived from sea urchin shells. Also, this invention includes a manufacturing method for such a composition; foods containing such a composition; and pharmaceutical compositions containing such a composition. There is no limitation on the species of the sea urchin material, e.g. "Bafun-uni" (Hemicentrotus pulcherrimus), "Murasaki-uni" (Anthocidaris crassispina), "Ezobafun-uni" (Strongylocentrotus intermedius), or "Aka-uni" (Pseudocentrotus depressus), can be used. To manufacture the calcium containing composition described in this invention, the soft content held in the sea urchin shells is initially removed. This can be done, for example, by crushing sea urchins and removing the flesh held in the shells. The shells are washed after the removal of the contents. Such a washing process can be done with water.

Then, the shells are baked at high temperature. The baking temperature is preferably 500 to 1500°C. The baking time is preferably 0.5 to 1.5 hours. For example, heating can be at 1000°C for 1 hour when a baking oven is used, or at 800°C for 1 hour when a vacuum oven is used. By such heating, calcium present in the sea urchin shells as calcium carbonate (CaCO₃) is converted to calcium oxide (CaO). After baking, such CaO can be converted, if necessary, into any useful formulation such as calcium carbonate, calcium hydroxide, calcium citrate, or calcium alginate. For example, conversion to calcium carbonate can be done by reacting carbon dioxide (CO₂) with the calcium oxide. It should be noted that there should not be any toxicity problem as calcium derived from sea urchin shells is obtained from an edible raw material.

Furthermore, foods, animal feeds, or pharmaceutical compositions containing this calcium containing composition derived from heated sea urchin shells of this invention can be made by, for example, mixing the calcium containing composition derived from baked sea urchin shells with citric acid and other formulation agents in conventional manner to form tablets or granules, or otherwise, or dissolving with other flavors to formulate for drinking use. The content of the calcium containing composition can be appropriately determined, but it is usually 10 to 50 weight % of the total amount of food or other end product. In this manner, high oral bioavailability of calcium can be achieved by taking the edible product containing the calcium derived from sea urchin shells of this invention. The above and other objects, features and advantages of the present invention will become clear from the following description of preferred embodiments thereof, taken in conjunction with the accompanying drawing.

In the drawing:

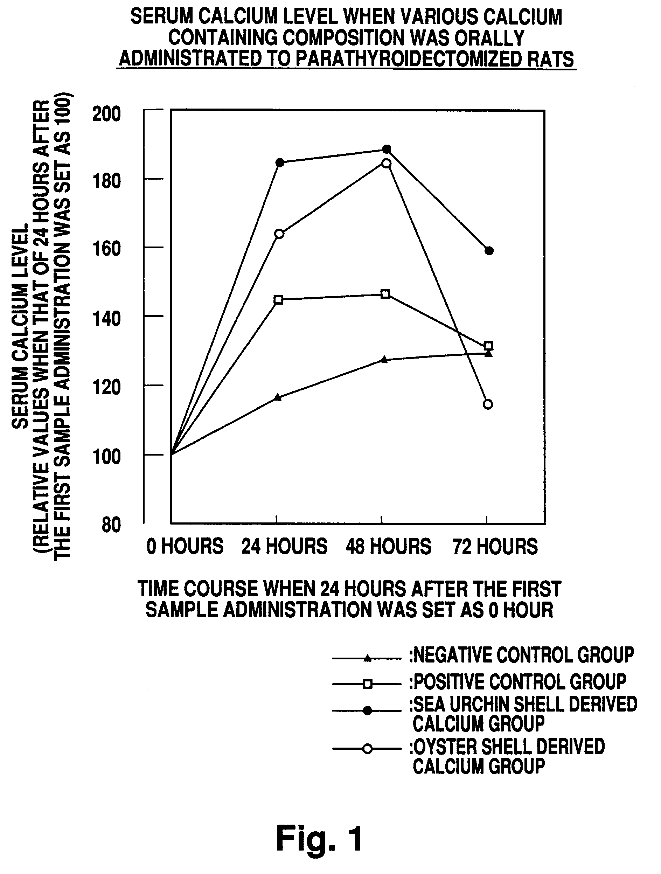

Fig. 1 indicates the serum calcium level when various calcium containing compositions were orally administered to parathyroidectomized rats (Serum calcium concentration 24 hours after the first administration of the samples was set at 100 and the relative value every 24 hours thereafter in each group was recorded. In the meantime, it was confirmed that the serum calcium level 24 hours after the first administration of the sample was as low as the level directly before the first administration).

### Example 1

"Bafun-uni" (Hemicentrotus pulcherrimus) and "Murasaki-uni" (Anthocidaris crassispina) were washed with water after the flesh portion was removed with a spatula. These sea urchin shells were baked at 1000°C for 1 hour in a oven. By such heating the calcium contained as calcium carbonate in the sea urchin shells was converted into calcium oxide. After heating, an appropriate amount of water was added, mixing to react to form calcium hydroxide. Table 1 indicates the result of the analysis of each component.

**Table 1**

| **Analytical Data of Sea Urchin Shell-Derived Calcium Containing Composition** | | |
|---|---|---|
| Components | Quantity | Analytical Methods |
| Phosphorus | 101 mg/100 g | Vanadato molybdate Spectrophotometry |
| Iron | 6.03 mg/100 g | Low phenanthroline Absorption Spectrophotometry |
| Calcium | 52.2% | Potassium Permanganate Titration |
| Sodium | 665 mg/100 g | Atomic Absorption Spectrometry |
| Potassium | 130 mg/100 g | Atomic Absorption Spectrometry |
| Magnesium | 2.14% | Atomic Absorption Spectrometry |
| Chlorine | 0.18% | Volhard's Method |
| Arsenic (as As₂O₃) | Not Detected (<0.55pm) | DDTC-Ag Absorption Spectrophotometry |
| Heavy Metals (as Pb) | 2.3 ppm | Sodium Sulfide Colorimetry |
| Copper | 2.1 ppm | Atomic Absorption Spectrometry |
| Zinc | 34.2 ppm | Atomic Absorption Spectrometry |
| Manganese | 1.5 ppm | Atomic Absorption Spectrometry |
| Sulfur | 0.05% | Barium Sulfate Gravimetry |

Thereafter, the characteristics of the calcium containing composition of this invention was compared with calcium containing compositions made from other raw materials. The comparison of the samples was done after the calcium included for example as calcium carbonate, in each raw material has been converted into calcium oxide by heating at 1000°C for 1 hour, and then had been again converted into calcium carbonate by adding carbon dioxide gas to make it edible. The results are shown in Table 2.

**Table 2**

| **Comparison of Characteristics of Various Calcium Containing Compositions** | | | | |
|---|---|---|---|---|
| Raw Materials | Appearance | Taste | Impurity | Yield(%) |
| Sea Urchin Shells | Pure White | Very Mild | No | 65 |
| Oyster Shells | Pale Gray | Sharp | Yes | 35 |
| Scallop Shells | Pale Yellow | Sharp | Yes | 40 |
| Corals | White and Hard | Sharp | Yes | 40 |
| Egg Shells | Pale yellow | Sharp | No | 45 |

As indicated by Table 2, the calcium containing composition derived from sea urchin shells provides a preferable appearance and taste, does not contain impurity and gives a high yield.

A comparison of oral bioavailability was also conducted between the calcium containing composition derived from sea urchin shells of this invention and other types of calcium containing compositions derived from other raw materials.

Positive control samples used in this experiment were sedimented calcium carbonate (6th Edition of Japanese Pharmacopeia) which is used as a therapeutic agent with more than 98.5% purity, calcium lactate, and calcium chloride (12th Revision of Japanese Pharmacopeia).

Rat intestine was used for testing bioavailability.

Specifically, rats were divided into groups of five animals, anaesthetized, their abdomens opened, the intestines occluded, and then blood was sampled from the intestinal vein. After that, 5 ml of aqueous solutions of 2% sedimented calcium carbonate, 2% calcium lactate, 2% calcium chloride, and 2% sea urchin shell derived calcium of this invention respectively at pH 2.0 were injected into the intestine. After injection, blood was sampled from the intestinal vein every 10 minutes, and the total serum calcium ion concentration was measured.

When 5 ml of aqueous solution of 2% sea urchin shell derived calcium containing composition at pH 2.0 was injected into the intestine, the serum calcium level started to increase 10 minutes later, and a significant increase was observed 30 minutes later. Surprisingly, the level of serum calcium ion induced by sea urchin shell derived calcium containing composition was 8.5 times higher than that by sedimented calcium carbonate, 4 times higher than that by calcium chloride, and 3 times higher than that by calcium lactate.

### Example 2

A calcium containing composition derived from sea urchin shells of this invention was administered to parathyroidectomized rats to compare the oral bioavailability of this composition to that of other forms of calcium containing composition. The "Sea urchin shell derived calcium containing composition" and "Oyster shell derived calcium containing composition" used here are identical to those used in Example 1. Parathyroidectomy was performed with several rats and a low calcium diet (a normal rat diet containing only 0.1% of calcium) was given for 156 hours to lower the blood calcium level. Twenty-four healthy rats (12 each of males and females) with sufficiently low blood calcium level were selected and divided into four groups of 6 animals (the same number of males and females in each group, respectively). These animals were fasted, and the first administrations of the samples were done 12 hours thereafter.

The following treatment was followed for the respective groups after that.
1) Negative Control Group: Saline (2 ml) injected into stomach as the first sample administration. Low calcium diet was given from 24 to 96 hours after that.
2) Positive Control Group: Saline (2 ml) solution of calcium carbonate (68.4 mg of calcium/kg rat weight was dissolved into the saline) was injected into the stomach as the first sample administration. The low calcium diet mentioned above containing calcium carbonate (68.4 mg calcium/kg rat weight per day was mixed in the diet) was given from 24 to 96 hours after that.
3) Sea Urchin Shell Derived Calcium Group: Saline solution (2 ml) of the sea urchin shell derived calcium containing composition (68.4 mg of calcium/kg rat weight was dissolved into the saline) was injected into the stomach as the first sample administration. The low calcium diet mentioned above containing the sea urchin shell derived calcium containing composition (68.4 mg calcium/kg rat weight per day was mixed in the diet) was given from 24 to 96 hours after that.
4) Oyster Shell Derived Calcium Group: Saline solution (2 ml) of the oyster shell derived calcium containing composition (68.4 mg of calcium/kg rat weight was dissolved into the saline) was injected into the stomach as the first sample administration. The low calcium diet mentioned above containing the oyster shell derived calcium containing composition (68.4 mg calcium/kg rat weight per day was mixed in the diet) was given from 24 to 96 hours after that.

A low calcium diet was provided to all four groups above between 6 and 24 hours after the first sample administration.

Blood was drawn from all animals of the above 4 groups immediately before the first sample administration (0 hour) and at 24, 48, 72, and 96 hours thereafter, and the serum calcium level was measured. The results are shown in Fig. 1 (24 hours after the first sample administration is set as 0 hour in this Figure. However, it was confirmed that the serum calcium level at 24 hours after the first sample administration came down to the same level as that at O hour.). As shown by this Figure, the increase of serum calcium level was higher in the order of sea urchin shell derived calcium group > oyster shell derived calcium group > positive control group > negative control group, and it was demonstrated that the sea urchin shell derived calcium containing composition is superior in oral bioavailability.

Faeces of all of the rats in all 4 groups were collected between the first sample administration and 24 hours thereafter and the calcium content was analyzed. The results are shown in the Table 3.

**Table 3**

| **Faecal Calcium Excretion When Various Calcium Containing Compositions were Orally Administered to Parathyroidectomized Rats** | |
|---|---|
| | Faecal Calcium Content (mg) |
| Negative Control | 0.881 |
| Positive Control | 1.624 |
| Sea Urchin Shell Derived Calcium | 0.475 |
| Oyster Shell Derived Calcium | 1.025 |

As indicated in this table, faecal calcium excretion was higher in the order of Positive Control > Oyster Shell Derived Calcium > Negative Control > Sea Urchin Shell Derived Calcium. The fact that faecal calcium excretion was lowest in the Sea Urchin Shell Derived Calcium Group means that the sea urchin shell derived calcium containing composition is absorbed from the intestine better when it is orally administered and excreted less into faeces. Specifically, it was confirmed that the sea urchin shell derived calcium containing compositions are superior in bioavailability compared to other forms of calcium containing composition when they are orally administered. In general, the dietary passage time in rats is said to be about 6 hours. Therefore, each sample injected into the stomach with saline at the first sample administration is regarded to be excreted to the faeces during the first 24 hours time period unless it is not administered at the intestine.

Normal rats and parathyroidectomized rats with lower serum calcium levels were used in Examples 1 and 2, respectively. However, both examples demonstrate the superior oral calcium bioavailability of sea urchin shell derived calcium containing compositions quantitatively. By these facts, it is evident that the urchin shell derived calcium containing compositions of this invention are useful in the treatment and prophylaxis of various diseases which require calcium supplementation such as hypocalcemia, osteoporosis, and renal osteodystrophy. Furthermore, taking into consideration the fact that sea urchin shell derived calcium containing compositions have better appearance and taste and contain low levels of impurity, as indicated in Example 1, it is evident that these compositions are extremely suitable for including in health foods and animal feeds for the purpose of calcium supply.

This invention therefor provides a calcium containing composition which is superior in oral bioavailability, has better taste, contains low impurity, and gives high yield from sea urchin shells that used to be discarded.

Such sea urchin shell derived calcium containing compositions are useful in the treatment or prophylaxis of diseases which require calcium supplementation such as hypocalcemia, osteoporosis, and renal osteodystrophy. Also, they are suitable for inclusion in health foods and animal feeds for the purpose of calcium supply.

While there have been shown what are considered to be the preferred embodiments of the invention, it will be manifest that many changes and modifications may be made therein without departing from the scope of the invention. It is intended, therefor, in the annexed claims, to cover all such changes and modifications as may fall within the true scope of the invention.

## Claims

1. A method of manufacturing a calcium containing composition including the baking of sea urchin shells.

2. The method of claim 1, wherein the baking temperature is 500 to 1500°C.

3. The method of claim 1 or 2, wherein the baking time is 0.5 to 1.5 hours.

4. The method of any one of claims 1 to 3, wherein calcium oxide is converted after baking into calcium carbonate, calcium citrate, calcium hydroxide, or calcium alginate.

5. A calcium containing composition obtainable by the method of any one of claims 1 to 4.

6. Foods containing the calcium containing composition of claim 5.

7. Animal feeds containing the calcium containing composition of claim 5.

8. A pharmaceutical composition containing the calcium containing composition of claim 5.

9. Use of the calcium containing composition of claim 5 in the manufacture of a pharmaceutical composition for the treatment or prophylaxis of a calcium deficiency disease.

10. The use of claim 9, wherein said calcium deficiency disease is hypocalcemia, osteoporosis or renal osteodystrophy.

11. Use of the calcium containing composition of claim 5 in the manufacture of foods or animal feeds.

## Patentansprüche

1. Verfahren zur Herstellung einer Calcium enthaltenden Zusammensetzung, umfassend das Backen von Seeigelschalen.

2. Verfahren nach Anspruch 1, wobei die Backtemperatur 500 bis 1500°C beträgt.

3. Verfahren nach Anspruch 1 oder 2, wobei die Backzeit 0,5 bis 1,5 Stunden beträgt.

4. Verfahren nach einem der Ansprüche 1 bis 3, wobei Calciumoxid nach dem Backen in Calciumcarbonat, Calciumcitrat, Calciumhydroxid oder Calciumalginat umgewandelt wird.

5. Calcium enthaltende Zusammensetzung, die durch ein Verfahren nach einem der Ansprüche 1 bis 4 erhältlich ist.

6. Nahrungsmittel, enthaltend die Calcium enthaltende Zusammensetzung nach Anspruch 5.

7. Futtermittel, enthaltend die Calcium enthaltende Zusammensetzung nach Anspruch 5.

8. Arzneimittel, enthaltend die Calcium enthaltende Zusammensetzung nach Anspruch 5.

9. Verwendung der Calcium enthaltenden Zusammensetzung nach Anspruch 5 für die Herstellung eines Arzneimittels zur Behandlung oder Prophylaxe einer Calciummangelerkrankung.

10. Verwendung nach Anspruch 9, wobei die Calciummangelerkrankung Hypocalcämie, Osteoporose oder renale Osteodystrophie ist.

11. Verwendung der Calcium enthaltenden Zusammensetzung nach Anspruch 5 für die Herstellung von Nahrungsmitteln oder Futtermitteln.

## Revendications

1. Procéde de fabrication d'une composition contenant du calcium comprenant la cuisson de coquilles d'oursins marins.

2. Procédé selon la revendication 1, dans lequel la température de cuisson est de 500 à 1 500°C.

3. Procédé selon la revendication 1 ou 2, dans lequel la durée de cuisson est de 0,5 à 1,5 heure.

4. Procédé selon l'une quelconque des revendications 1 à 3, dans lequel l'oxyde de calcium est transformé après cuisson en carbonate de calcium, citrate de calcium, hydroxyde de calcium ou alginate de calcium.

5. Composition contenant du calcium pouvant être obtenue avec le procédé de l'une quelconque des revendications 1 à 4.

6. Aliments contenant la composition contenant du calcium selon la revendication 5.

7. Aliments d'animaux contenant la composition contenant du calcium selon la revendication 5.

8. Composition pharmaceutique contenant la composition contenant du calcium selon la revendication 5.

9. Utilisation de la composition contenant du calcium selon la revendication 5 dans la fabrication d'une composition pharmaceutique pour le traitement et la prophylaxie d'une maladie de carence en calcium.

10. Utilisation selon la revendication 9, dans laquelle ladite maladie de carence en calcium est l'hypocalcémie, l'ostéoporose ou l'ostéodystrophie rénale,

11. Utilisation de la composition contenant du calcium selon la revendication 5 dans la fabrication d'aliments ou d'aliments pour animaux.
